# EUROPEAN PATENT SPECIFICATION

(11) **EP 1 250 925 B1**
(45) Date of publication and mention of the grant of the patent: **15.06.2005**
(21) Application number: 02252717.0
(22) Date of filing: 17.04.2002
(51) Int. Cl.: A61K 31/4178, A61K 9/12, A61K 47/40, A61P 1/08

(54) **Nasal spray containing ondansetron hydrochloride**
Nasenspray enthaltend Ondansetron-hydrochlorid
Pulvérisateur nasal contenant du chlorhydrate d'ondansétron

(30) Priority: 20.04.2001 KR 2001021573; 11.01.2002 KR 2002001758
(43) Date of publication of application: 23.10.2002
(73) Proprietor: AHN-Gook Pharma Co., Ltd., Yongdungpo-ku, Seoul (KR)
(72) Inventor: Auh, Jin, Kangnam-ku, Seoul (KR); Kim, Chang Hwan, Ansan-si, Gyeonggi-do (KR); Chun, In Koo, Seocho-ku, Seoul (KR)
(74) Representative: Bassil, Nicholas Charles

(56) References cited:
- WO-A-00/74651
- GB-A- 2 231 265
- HUSSAIN ANWAR A ET AL: "Nasal absorption of ondansetron in rats: An alternative route of drug delivery." CANCER CHEMOTHERAPY AND PHARMACOLOGY, vol. 45, no. 5, May 2000 (2000-05), pages 432-434, XP002256140 ISSN: 0344-5704

## Description

### FIELD OF THE INVENTION

The present invention relates to an antiemetic nasal spray composition, particularly an antiemetic nasal spray composition which is easily administered and rapidly absorbed through the nasal mucosa to exert prompt antiemetic effect.

### BACKGROUND OF THE INVENTION

The side effects, such as nausea and vomiting, being usually encountered in the chemotherapy can lead to dehydration, severe metabolic imbalance and malnutrition in patients. Accordingly, the patient tends to refuse the chemotherapy itself. Therefore, a prophylaxis of nausea and vomiting induced by anticancer drugs could improve the effect of chemotherapy. To prevent the chemotherapy-induced nausea and vomiting, 1,2,3,9-tetrahydro-9-methyl-3-[(2-methyl-1H-imidazol-1-yl)methyl]-4H-carbazol-4-one hydrochloride dihydrate (hereafter, refer to "ondansetron hydrochloride") can be used.

Ondansetron hydrochloride has been usually used by oral and injectable administration under the brand name of "Zofran^{R}" to suppress nausea and vomiting as a selective antagonist at 5-HT₃ receptor.

However, a conventional oral administration has good absorption, but extensively metabolized by the liver (first-pass hepatic metabolism). It should be also administered 30 minutes before chemotherapy, and the orally administered anticancer drug tends to be discharged by vomiting. Also, intravenous administration renders rapid effects to a patient, but the onset of effects is so rapid that it causes undesirable effects. In addition, it gives a local pain, and may cause an unexpected accident when it is not perfectively prepared.

Korean Patent Application Laid-open No. 2001-14479 discloses a technology to solve the above problems. This laid-open application provides a composition containing ondansetron hydrochloride of which dermal application is feasible. Since this composition contains a hydrophilic organic solvent, skin-permeation enhancer and water, the composition can alleviate side effects and maintain an effective blood concentration of antiemetic drug.

However, the composition has a few disadvantages that it cannot rapidly exert an effect, because the active ingredient of the composition is absorbed into the systemic blood concentration through the skin, and generally it takes a long lag time to reach the steady state blood level. Therefore, the transdermal delivery formulation has to be produced in a form of patch and attached on to the skin for a long time. This also causes a skin damage due to a long term attachment thereto. In addition, it is disadvantageous that antiemetic drug can not be timely administered when it is required.

### SUMMARY OF THE INVENTION

The object of the present invention is to provide a nasal delivery composition which exerts rapid and effective antiemetic effect when it is administered.

The another object of the present invention is to provide an antiemetic composition which can be simply and readily administered.

The still another object of the present invention is to provide an antiemetic composition which can prevent the administered antiemetic drug from being discharged due to vomiting.

The still another object of the present invention is to provide an antiemetic composition which has no side effects such as local pains, skin rash and the like.

The still another object of the present invention is to provide an antiemetic composition which can be maintained in a solution phase for a long time so that the effective component may be nasally administered by spraying.

### DESCRIPTION OF THE INVENTION

The antiemetic composition of the present invention comprises
(1) 2-8 parts by weight of ondansetron hydrochloride as an antiemetic drug; and
(2) 100 parts by weight of base materials for nasal administration consisting of (i) 70-85% by weight of water, (ii) 5-15% by weight of polyethylene glycol (hereafter refer to "PEG"), (iii) 0.005-0.02% by weight of benzalkonium chloride (hereafter refer to "BC") and (iv) 7-20% by weight of one solubilizer selected from sulfobutyl ether β-cyclodextrin sodium salt (hereafter refer to "SBCD"), dimethyl-β-cyclodextrin (hereafter refer to "DMCD") and 2-hydroxypropyl-β-cyclodextrin (hereafter refer to "2HPβ CD"), the total amount of the above base materials being 100% by weight.

The present invention will be explained in detail as follows.

Since a nasal cavity has a relatively high surface area of 150-180cm² and a thin mucosa of 2-4 mm, it relatively rapidly absorbs most of water soluble drugs. Some of the major advantages offered by the nasal administration are as follows:
(1) Dose of a drug can be reduced, because a drug is rapidly absorbed and its bioavailability is high.
(2) Onset of therapeutic action is fast.
(3) Hepatic first pass metabolism can be avoided.
(4) A metabolism in gastrointestinal tract can be avoided.
(5) Irritation of the mucosa in the gastrointestinal tract can be avoided.
(6) There is little risk to be administered in overdose.
(7) There is little risk to be infected because of non-invasiveness.
(8) It is convenient to use a drug, and the drug can be administered without assistance by other person.
(9) Patient compliance is improved.
(10) Nasal administration can be beneficial to the conventional administration.
(11) The risk of infectious disease occurrence can be reduced.

Although the nasal administration has such many advantages, the formulation to be intranasally administered should be prepared in a solution and should not form precipitates even during the longer period of storage. The higher permeation across the nasal mucosa can be easily achieved when the active ingredient concentration should be high in solution phase. However, it is difficult to meet the above two requirements at the same time.

The present inventors have fervently studied for solving these difficulties. As a result, they have found that the ondansetron hydrochloride nasal spray formulation could be prepared in a higher concentration and did not form any precipitates. Ondansetron hydrochloride was stable in the offered nasal formulation. The permeation rate of the active ingredient through the nasal mucosa was remarkably increased by adequately selecting and adjusting the additives and their amount in the nasal composition used as base material of ondansetron hydrochloride.

PEG used in the present invention maintains the surface of the nasal mucosa to be wet so as to facilitate permeation of the active ingredient through the nasal mucosa. The amount of the PEG is preferably 5-15% by weight on the basis of the amount of the base material. When the amount of the PEG is less than 5 % by weight, it unsatisfactorily wets nasal mucosa. And when the amount of the PEG is more than 15 % by weight, it may not improve the wetting of nasal mucosa, rather lowers the physical stability on storage of the composition and the transnasal permeation rate of the active ingredient. Further, when it is nasally sprayed, it may form precipitates, and thus plug the orifice of the nozzle.

The BC used in the present invention is a preservative to prevent the composition from the microbial contamination, and is used in an amount of preferably 0.005-0.02 % by weight based on the weight of the base material. When the amount of the BC is less than 0.005 % by weight, its preservative activity is lowered. And when the amount of the BC is more than 0.02 % by weight, it can cause an irritation of nasal mucosa.

Examples of the solubilizers used in the present invention include SBCD, DMCD and 2HPβ CD. One of them is preferably used in an amount of 7-20 % by weight based on the base material. When the amount of the solubilizer is less than 7 % by weight, the enhancement of solubility of ondansetron hydrochloride as an active ingredient is not sufficient, and the composition tends to be precipitated. And when the amount of the solubilizer is more than 20 % by weight, the transnasal permeation of the active ingredient is lowered. The SBCD of the above solubilizers has most excellent solubilizing effect.

According to the present invention, the amount of the water as an aqueous vehicle is preferably 70-85% by weight on the basis of the base material. When the amount of water is less than 70 % by weight, the ultimately obtained composition tends to be precipitated. And when the amount of water is more than 85 % by weight, the solubility of ondansetron hydrochloride as an active ingredient will be lowered by using the reduced amount of solubilizer, or the moistening effect on the mucosal surface will be lowered by using the reduced amount of humectant (PEG). The composition for nasal administration according to the present invention has to be maintained in a solution phase for a long time at room temperature or in a cold place contrary to the formulations for oral or transdermal administration. Accordingly, the composition according to the present invention can not be used when it is precipitated.

Ondansetron hydrochloride as an active ingredient is dissolved up to 8 parts by weight based on 100 parts by weight of the base material of the above composition. The higher the concentration of the active ingredient in the solution spray composition is, the more the nasal mucosal permeation is enhanced. However, considering the long-term storage stability of the formulation, it is preferable to solubilize not more than 6 parts by weight of the active ingredient. If necessary, not more than 2% by weight of a water-soluble chitosan may be added to the composition in order to increase the adhesiveness of the composition to the nasal mucosa. Further, pH of the composition is preferably adjusted to a weak acidity of pH 4.0-6.0 so as to increase the chemical stability of the active ingredient and aid to prevent the growth of microorganisms. Examples of the pH adjusting agent include hydrochloric acid, phosphoric acid, citric acid, tartaric acid and the like.

The composition according to the present invention is administered by spraying it to the nasal mucosa, and rapidly exerts its antiemetic effect within 30 minutes after nasal administration. In addition, since the bioavailability of the active ingredient is higher in nasal administration than that in oral administration, the desired effect can be attained by using even a reduced dose of the active ingredient.

### EXAMPLES

The present invention is described in more detail by Examples and Comparative Examples.

### Formulation Example

### (Preparation of Antiemetic Nasal Spray Composition)

10% by weight of PEG 300, 10% by weight of SBCD, 0.01% by weight of BC and the balance of water were mixed to obtain the base material. 4 parts by weight of ondansetron hydrochloride are added and dissolved in 100 parts by weight of the obtained base material to give an antiemetic nasal spray composition.

### Example 1

### (Test for Storage Physical Stability)

The composition obtained in the above Formulation Example was adjusted to a weak acidity of pH 4.58 in order to increase the chemical stability of the active ingredient and the antimicrobial preservation. This composition was stored in a refrigerator maintained at a temperature of 4ºC to observe the formation of precipitates. Even after 30 days, any precipitation was not observed. This result indicates that the composition of the present invention can be stored at room temperature for a long term.

### Example 2

### (Test for Intranasal Stability of Active Ingredient)

The fresh nasal mucosa excised from a rabbit (body weight 3.0 kg, New Zealand White female rabbit) was mounted to the Valia-Chien permeation system maintained at a constant temperature of 37ºC. Each of the donor and receptor half-cells was filled with each 3.5mL of saline, maintained at the constant temperature for 4 hours while stirring with a magnetic star-head bar, and then both extracts from the half-cells were combined and used as nasal mucosa extracts.

Ondansetron hydrochloride (200 µg/mL) was added to the obtained nasal mucosa extract, and then maintained at the temperature of 37ºC for 4 hours while gently shaking in order to confirm the physiochemical or enzymatic stability of ondansetron hydrochloride in a nasal cavity. The results are shown in below Table 1.

**Table 1**

| Time (hr) | Residual Concentration (µg/mL)* |
|---|---|
| 0 | 198.6 ± 1.3 |
| 1 | 199.0 ± 5.2 |
| 2 | 203.5 ± 3.0 |
| 3 | 196.9 ± 0.6 |
| 4 | 197.9 ± 9.6 |

| | |
|---|---|
| note: "*" means the average (standard deviation, n=3) | |

The results in Table 1 show that ondansetron hydrochloride was not nearly degraded in nasal mucosa extract for 4 hours. This means that ondansetron hydrochloride is stable at the site of administration and absorption, namely nasal mucosa.

### Example 3

### (Test for Permeation of Active Ingredient)

Permeation rates of ondansetron hydrochloride through nasal, duodenal, colonic and rectal mucosae of a rabbit (body weight 3.0 kg, New Zealand White female rabbit) were determined. The results are shown in below Table 2. As shown in Table 2, the permeation of ondansetron hydrochloride in nasal mucosa occurs very rapidly. The permeation rate of 10 minutes reached 30.4 µg/cm², and the permeation rate through nasal mucosa at 30 minutes was 7.2, 7.5 and 15.8 times higher than those through the duodenal, colonic and rectal mucosae, respectively.

**Table 2**

| Time (hr) | Cumulative Amounts (µm/cm²)* | | | |
|---|---|---|---|---|
| | Nasal mucosa | Duodenal mucosa | Colonic mucosa | Rectal Mucosa |
| 0.167 | 30.4 ± 3.2 | -** | - | - |
| 0.333 | 68.2 ± 4.7 | - | - | - |
| 0.5 | 106.8 ± 14.9 | 14.8 ± 3.5 | 14.3 ± 2.2 | 6.7 ± 0.1 |
| 1.0 | 180.5 ± 12.4 | 35.4 ± 8.2 | 36.5 ± 7.8 | 9.6 ± 0.9 |
| 1.5 | 261.5 ± 15.1 | - | - | - |
| 2.0 | 289.3 ± 23.5 | 94.6 ± 23.9 | 112.1 ± 14.6 | 19.3 ± 5.8 |
| 3.0 | 414.6 ± 22.6 | - | - | - |
| 4.0 | 559.4 ± 28.2 | 280.1 ± 43.2 | 294.8 ± 14.7 | 54.1 ± 6.7 |
| 6.0 | - | 453.1 ± 36.3 | 438.9 ± 27.2 | 164.2 ± 9.2 |

| | | | | |
|---|---|---|---|---|
| note: "*" means the average (standard deviation, n=3) | | | | |
| "*'*" means that the amounts were not determined. | | | | |

### Example 4

### (Test for Effect of Permeation Enhancers)

As permeation enhancers, each 1% by weight of sodium caprate, SGC (sodium glycocholate), ammonium glycyrrhizinate, sodium deoxycholate, lauroylsarcosine and each of 0.1% by weight of EDTA and EDTA disodium were added to the antiemetic nasal spray composition in order to increase the permeation of the active ingredient. However, the permeation enhancers were not dissolved in the composition except for the mixture of 1% by weight of SGC and 0.1% by weight of EDTA disodium. As shown also in Table 3, the permeation rate of active ingredient from the composition containing permeation enhancer was rather lower than that of the composition (MV) containing no permeation enhancer. This means that the composition of the present invention has a high nasal permeability even without permeation enhancer.

**Table 3**

| Time (hr) | Cumulative Amounts (µm/cm²)* | | |
|---|---|---|---|
| | MV** | MV** + SGC 1% | MV** + EDTA disodium 0.1% |
| 0.167 | 14.2 ± 6.8 | 4.0 ± 0.6 | 3.4 ± 0.1 |
| 0.333 | 51.9 ± 15.7 | 11.7 ± 0.6 | 9.9 ± 2.0 |
| 0.5 | 112.6 ± 19.3 | 27.8 ± 1.9 | 22.9 ± 4.6 |
| 1.0 | 306.8 ± 37.4 | 173.1 ± 2.0 | 115.3 ± 15.4 |
| 1.5 | 556.0 ± 49.7 | 340.0 ± 25.5 | 233.5 ± 21.2 |
| 2.0 | 808.2 ± 67.5 | 563.2 ± 62.0 | 398.1 ± 25.1 |
| 3.0 | 1368.0 ± 114.5 | 1001.0 ± 140.6 | 813.5 ± 70.7 |
| 4.0 | 1888.7 ± 177.0 | 1460.9 ± 270.6 | 1250.2 ± 138.6 |

| | | | |
|---|---|---|---|
| note: "*" means the average (standard deviation, n=3) | | | |
| "**" : MV=Antiemetic Composition Obtained in Formulation Example | | | |

### Example 5

### (Comparison of Bioavailability for Active Ingredient)

Twenty four (24) white rats (body weight 250-300 g, Sprague-Dawley male rats) were classified into 3 groups, namely 6, 11 and 7 rats. After nasal, intravenous and oral administration to each group of rats, the bioavailability according to the different routes of administration was compared.

Ondansetron hydrochloride was dissolved in the composition obtained in Formulation Example for nasal administration, in 0.9% saline for intravenous administration, and in purified water for oral administration as vehicles, respectively. Each 2 mg/kg of the solutions was administered thereby. The blood samples were collected at the predetermined time intervals to analyze the concentration of ondansetron hydrochloride in the plasma. The bioavailibity parameters were calculated from the assay results. The results are shown in below Table 4.

Ondansetron hydrochloride was rapidly absorbed through nasal route. The peak plasma concentration (Cₘₐₓ) of ondansetron hydrochloride was found to be 49.4 ± 18.7(ng/mL), and the time to reach the peak concentration (Tₘₐₓ) was 9.4 minutes. On the contrary to this, the Cₘₐₓ in oral administration comes up to 29.7 ± 10.6 ng/mL which is 1.7 times lower than that in nasal administration. Tₘₐₓ in oral administration was 11.8 minutes. Area under the plasma concentration-time curve (AUC₀₋₂ₕᵣ) in nasal administration was 53.2 ± 9.5 ng·hr/mL, AUC₀₋₂ₕᵣ in oral administration was 24.9 ± 12.6 ng·hr/mL and AUC₀₋₂ₕᵣ in intravenous administration was 51.1 ± 7.4 ng·hr/mL. Accordingly, AUC₀₋₂ₕᵣ in nasal administration was 2.1 times higher than that in oral administration and was almost same as that in intravenous administration.

| Route of administration | Number of animals | Cₘₐₓ (ng/mL)* | Tₘₐₓ (min)* | AUC₀₋₂ₕᵣ (ng.hr/mL)* | AUC_{oral.nasal}/AUVᵢᵥ* |
|---|---|---|---|---|---|
| IV injection | 6 | -** | -** | 51.1 ± 7.4 | -** |
| Oral | 11 | 29.7 ± 10.6 | 11.8 ± 1.8 | 24.9 ± 12.6 | 0.49 |
| Nasal | 7 | 49.4 ± 18.7 | 9.4 ± 1.8 | 53.2 ± 9.5 | 1.04 |

| | | | | | |
|---|---|---|---|---|---|
| note) "*" means the average (standard deviation). | | | | | |
| "**" means the parameter which cannot be detected. | | | | | |

### Example 6

The same procedure as defined in Formulation Example was conducted except that DMCD was used instead of SBCD to obtain the antiemetic composition. The experiments for storage physical stability, permeability, chemical stability and the like for the obtained composition were conducted. The results were similar to those in the above examples where SBCD was used.

### Example 7

The same procedure as defined in Formulation Example was conducted except that 2HPβ CD was used instead of SBCD to obtain the antiemetic composition. The experiments for storage physical stability, permeability, chemical stability and the like for the obtained composition were conducted. The results were similar to those in the above examples where SBCD was used. However, the solubility of ondansetron hydrochloride as an active ingredient was somewhat low comparing to the composition containing about 3% by weight of SBCD .

### Comparative Example 1

The same procedure as defined in Formulation Example was conducted except that the amount of SBCD was reduced to 5% by weight to obtain the antiemetic composition for nasal administration. The same experiment as Example 1 for storage stability for the obtained composition was conducted. However, precipitates began to be formed after 7 days.

### Comparative Example 2

The same procedure as defined in Formulation Example was conducted except that the amount of SBCD was increased to 25% by weight to obtain the antiemetic composition for nasal administration. The same experiment as Example 5 for comparing the bioavailability for active ingredient for the obtained composition was conducted. As a result, ondansetron hydrochloride was slowly absorbed through nasal mucosa than that in Example 5. The peak plasma concentration (Cₘₐₓ) was 36.2 ± 10.7 ng/mL in 9.8 minutes. Accordingly, it was confirmed that the absorption of drug is retarded since SBCD entraps ondansetron hydrochloride as an active ingredient, when SBCD is used in excessive amount.

### EFFECTS OF THE INVENTION

The antiemetic composition according to the present invention is feasible for nasal administration, and rapidly exerts its antiemetic effect within 30 minutes upon administering it to a human being. In addition, since bioavailability of ondansetron hydrochloride as an active ingredient is highly improved, the desired effect can be attained even by administering relatively small amount of the active ingredient. Furthermore, the antiemetic composition according to the present invention can completely avoid the discharge of the active ingredient from stomach in oral administration and the unfavorable effects such as local pains, skin rash and the like in intravenous of intramuscular administration.

## Claims

1. Antiemetic nasal spray composition comprising
(1) 2-8 parts by weight of ondansetron hydrochloride as an antiemetic drug; and
(2) 100 parts by weight of base material for nasal administration consisting of (i) 70-85% by weight of water, (ii) 5-15% by weight of polyethylene glycol, (iii) 0.005-0.02% by weight of benzalkonium chloride and (iv) 7-20% by weight of one solubilizer selected from sulfobutyl ether β-cyclodextrin sodium salt, dimethyl-β-cyclodextrin and 2-hydroxypropyl-β-cyclodextrin, the total amount of the above base material being 100% by weight.

2. Antiemetic nasal spray composition according to Claim 1, **characterized in that** not more than 2% by weight of chitosan is further added in order to increase the adhesiveness of the composition to the nasal mucosa.

3. Antiemetic nasal spray composition according to Claim 1, **characterized in that** pH 4.0-6.0 is maintained by adding a pH adjusting agent.

4. Antiemetic nasal spray composition according to Claim 1, **characterized in that** nasal administration is conducted by a spray through a fine nozzle.

5. Antiemetic nasal spray composition according to Claim 3, **characterized in that** pH adjusting agent is hydrochloric acid, phosphoric acid, citric acid or tartaric acid.

6. A composition as defined in any one of claims 1 to 3 or 5 for use in the treatment or prevention of nausea and/or vomiting.

7. The use of a composition as defined in any one of claims 1 to 3 and 5 in the preparation of an antiemetic nasal spray for the treatment or prevention of nausea and/or vomiting.

## Patentansprüche

1. Antiemetische Nasensprayzusammensetzung, welche Folgendes enthält:
(1) 2-8 Gewichtsteile Ondansetronhydrochlorid als antiemetischer Arzneistoff; und
(2) 100 Gewichtsteile Grundmaterial für die nasale Verabreichung bestehend aus (i) 70-85 Gew.-% Wasser, (ii) 5-15 Gew.-% Polyethylenglykol, (iii) 0,005-0,02 Gew.-% Benzalkoniumchlorid und (iv) 7-20 Gew.-% eines Lösungsverbesserers ausgewählt aus Sulfobutylether-β-Cyclodextrin Natriumsalz, Dimethyl-β-Cyclodextrin und 2-Hydroxypropyl-β-Cyclodextrin, wobei die Gesamtsumme des obengenannten Grundmaterials 100 Gew.-% ist.

2. Antiemetische Nasensprayzusammensetzung nach Anspruch 1, **dadurch gekennzeichnet, dass** nicht mehr als 2 Gew.-% Chitosan zusätzlich hinzugefügt sind, um das Adhäsionsvermögen der Zusammensetzung an die Nasenschleimhaut zu erhöhen.

3. Antiemetische Nasensprayzusammensetzung nach Anspruch 1, **dadurch gekennzeichnet, dass** pH 4,0-6,0 durch Hinzufügen eines pH-regulierenden Agens aufrechterhalten ist.

4. Antiemetische Nasensprayzusammensetzung nach Anspruch 1, **dadurch gekennzeichnet, dass** die nasale Verabreichung durch ein Spray über eine feine Düse erfolgt.

5. Antiemetische Nasensprayzusammensetzung nach Anspruch 3, **dadurch gekennzeichnet, dass** das pH-regulierende Agens Salzsäure, Phosphorsäure, Zitronensäure oder Weinsäure ist.

6. Zusammensetzung wie in einem der Ansprüche 1 bis 3 oder 5 definiert zur Verwendung bei der Behandlung oder Verhinderung von Übelkeit und/oder Erbrechen.

7. Verwendung einer Zusammensetzung wie in einem der Ansprüche 1 bis 3 oder 5 definiert bei der Herstellung eines antiemetischen Nasensprays zur Behandlung oder Verhinderung von Übelkeit und/oder Erbrechen.

## Revendications

1. Composition antiémétique pour pulvérisation nasale comprenant
(1) 2-8 parties en poids de chlorhydrate d'ondansétron sous forme d'un médicament antiémétique; et
(2) 100 parties en poids d'une substance de base pour une administration par voie nasale constituée de (i) 70-85% en poids d'eau, (ii) 5-15% en poids de polyéthylèneglycol, (iii) 0,005-0,02% en poids de chlorure de benzalkonium et (iv) 7-20% en poids d'un solubilisant choisi parmi le sel sodique de la sulfobutyléther-β-cyclodextrine, la diméthyl-β-cyclodextrine et la 2-hydroxypropyl-β-cyclodextrine, la quantité totale de la substance de base susmentionnée étant de 100% en poids.

2. Composition antiémétique pour pulvérisation nasale selon la revendication 1, **caractérisée en ce que** 2% en poids au maximum de chitosane sont en outre ajoutés afin d'augmenter l'adhérence de la composition à la muqueuse nasale.

3. Composition antiémétique pour pulvérisation nasale selon la revendication 1, **caractérisée en ce qu'**un pH de 4.0-6.0 est maintenu par l'addition d'un agent d'ajustement du pH.

4. Composition antiémétique pour pulvérisation nasale selon la revendication 1, **caractérisée en ce que** l'administration par voie nasale est réalisée par une pulvérisation via une mince canule.

5. Composition antiémétique pour pulvérisation nasale selon la revendication 3, **caractérisée en ce que** l'agent d'ajustement du pH est l'acide chlorhydrique, l'acide phosphorique, l'acide citrique ou l'acide tartrique.

6. Composition telle que définie selon l'une quelconque des revendications 1 à 3 ou 5 pour une utilisation dans le traitement ou la prévention des nausées et/ou des vomissements.

7. Utilisation d'un composition telle que définie selon l'une quelconque des revendications 1 à 3 et 5 dans la préparation d'une pulvérisation nasale antiémétique pour le traitement ou la prévention des nausées et/ou des vomissements.
